(19) Europäisches Patentamt
European Patent Office
Office européen des brevets

(11) Publication number: 0 177 080 B1

(12) # EUROPEAN PATENT SPECIFICATION

(45) Date of publication of patent specification: 17.11.88

(51) Int. Cl.[4]: C 07 D 311/56 // A01N43/16, A61K31/37

(21) Application number: 85201436.4

(22) Date of filing: 10.09.85

(54) Process for preparing 4-hydroxycoumarin derivatives.

(30) Priority: 26.09.84 GB 8424317

(43) Date of publication of application: 09.04.86 Bulletin 86/15

(45) Publication of the grant of the patent: 17.11.88 Bulletin 88/46

(84) Designated Contracting States: AT BE CH DE FR GB IT LI LU NL SE

(56) References cited:
GB-A-2 126 578

CHEMICAL ABSTRACTS, vol. 83, no. 13, September 29, 1975, Columbus, Ohio, USA, M.R. HADLER; R.S. SHADBOLT "Novel 4-hydroxycoumarin anticoagulants active against resistant rats.", p. 179, column 2, abstract no. 109 770r

CHEMICAL ABSTRACTS, vol. 86, no. 15, April 11, 1977, Columbus, Ohio, USA, WARD, BLENKINSHOP AND CO. LTD. (ENGL.). "Synthesis of tetrahydronaphthyl coumarins.", p. 484, column 1, abstract no. 106 300p

CHEMICAL ABSTRACTS, vol. 85, no. 15, October 11, 1976, Columbus, Ohio, USA, R.S. SHADBOLT; D.R. WOODWARD; P.J. BIRCHWOOD "Synthesis of some tetrahydronaphthyl- and flavanyl-coumarins", p. 447, column 1, abstract no. 108 487h

(73) Proprietor: SHELL INTERNATIONALE RESEARCH MAATSCHAPPIJ B.V.
Carel van Bylandtlaan 30
NL-2596 HR Den Haag (NL)

(72) Inventor: Entwistle, Ian David
44 Woodside Garden
Sittingbourne Kent (GB)
Inventor: Boehm, Peter
4 Netley Close Vinters Park
Maidstone Kent (GB)

(74) Representative: Hunter, Keith Roger Ian et al
4 York Road
London SE1 7NA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 177 080 B1

Courier Press, Leamington Spa, England.

**Description**

This invention relates to a process for preparing certain 4-hydroxycoumarin derivatives.

US Patent No. 3,957,824 (and the corresponding UK Patent Specification No. 1,458,670) discloses a class of 4-hydroxycoumarin derivatives having anti-coagulant properties, which are useful as rodenticides or in human medicine, of general formula

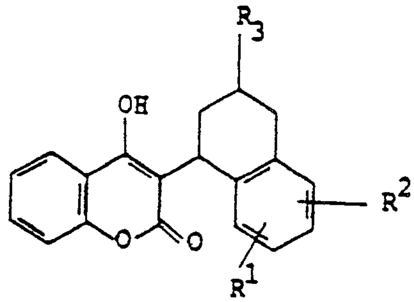

(I)

wherein $R^1$ and $R^2$ are the same or different and are hydrogen or halogen atoms, preferably chlorine or bromine, or alkyl or alkoxy groups, preferably having up to 6 carbon atoms, $R^3$ is an aryl group having the formula

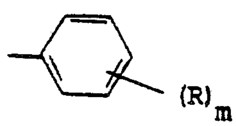

where m is 1 or 2, and R is the same or different and is a halogen atom, a straight or branched chain alkyl or alkoxy group, preferably containing at least 2, more preferably from 5 to 12 carbon atoms, a cycloalkyl, preferably cyclohexyl group, an aralkyl, preferably α-aralkyl group, a phenyl or a phenoxy group, a halogeno, preferably para halogeno, substituted derivative thereof. The halogen atom or atoms are preferably chlorine or bromine. Where m is 1, R is preferably in the para position and when m is 2 one of the R groups is preferably in the para position. Preferably $R^3$ contains at least 1 but not more than 3 and optimally not more than 2 halogen atoms.

The above compounds of formula I are prepared by condensing 4-hydroxy coumarin (II) with compounds of general formula III without a solvent or in an organic solvent such as acetic acid in the presence of a dehydrating agent such as sulphuric acid or by condensing 4-hydroxy coumarin (II) with a compound of general formula IV with or without the use of a solvent.

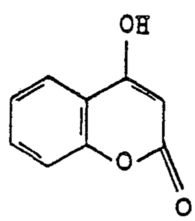

(II)

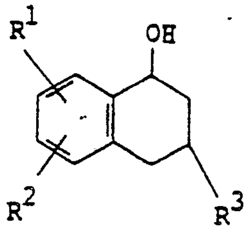

(III)

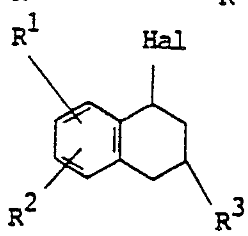

(IV)

$R^1$, $R^2$ and $R^3$ have the meanings given previously and Hal is halogen, preferably chlorine or bromine.

Compounds of structure IV may be prepared from compounds of general structure III by treatment with reagents such as phosphorus tribromide, phosphorus trichloride or thionyl chloride in an inert solvent such as methylene chloride, chloroform or carbon tetrachloride.

The lowest temperature employed in such a preparation is in condensation of such compounds of formulae II and III in acetic acid at 110°C. When no solvent is present, condensation of compounds of formulae II and III is typically effected at 160 to 170°C. Reaction of compound of formulae II and IV is typically at 130 to 140°C (for these latter temperature ranges see Shadbolt *et al* J. C. S. Perkin 1, (1976) No. 11, pages 1190 to 1195).

Two specific compounds of formula I above are that wherein $R^1$ and $R^2$ are both hydrogen and $R^3$ is a 4-phenylphenyl group (difenacoum) and that wherein $R^1$ and $R^2$ are both hydrogen and $R^3$ is a 4-(4-bromophenyl)phenyl group (brodifacoum).

GB—A—2 126 578, and the corresponding EP—A—98 629, discloses a further class of 4-hydroxycoumarin derivatives having anticoagulant properties, particularly as rodenticides. In their broadest definition, these compounds have the formula

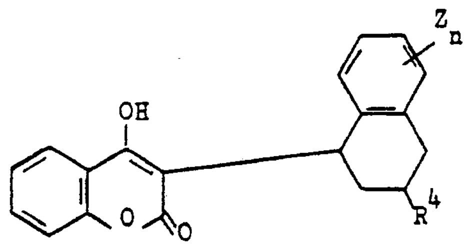

(V)

in which Z represents a halogen atom, preferably a chlorine atom, and n is 0, 1 or 2 and $R^4$ represents either (1) a grouping which comprises a phenylene radical attached directly or indirectly to the tetralin ring and having in the para position (with respect to such attachment) an electron-withdrawing atom or group whose rotational volume substantially does not exceed that of a phenyl group and which forms together with said phenylene radical a polarisable structure, or (2) a grouping selected from:

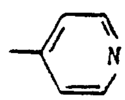 , 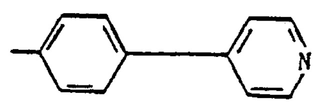

and 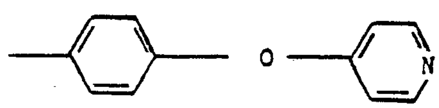

or (3) a grouping which comprises a phenylene radical attached directly to the tetralin ring and having in the para position (with respect to such attachment) a substituted furanyl or thiophenyl radical attached thereto directly or through oxygen and/or methylene, said furanyl or thiophenyl radical having an electron-withdrawing atom or group as a substituent in a position forming with the furanyl or thiophenyl radical a polarisable structure, said atom or group having a rotational volume which substantially does not exceed that of a phenyl group.

In the above formula V, $R^4$ is preferably more specifically defined as a group selected from

(a) 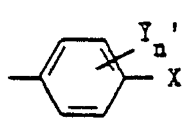

where X represents a halogen atom or a group CN, $NO_2$, $SO_2R^5$, $CF_3$, $OCF_3$, $COOR^5$ or $COR^5$, where $R^5$ is a $C_{1-6}$ alkyl group, n' is 0, 1 or 2 and Y is a fluorine or chlorine atom, provided that when X is halogen, n' is 1 or 2;

(b) 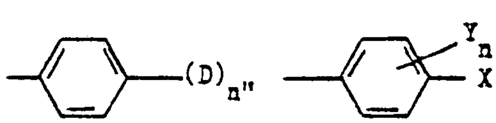

where X, n' and Y are as defined above, n'' is 0 or 1 and D is an oxygen atom or a group $—O—(CH_2)_m—$, $—(CH_2)_m—O—$, $—O—(CH_2)_m—O—$, $—(CH_2)_m—O—(CH_2)_p$, $(CH_2)—_m$, or —CH=CH—, or a sulphur analogue thereof, wherein m is in the range 1 to 6 and p is in the range 1 to 6, provided that when D is an oxygen

atom and X is halogen; n' is 1 or 2, and when D is —CH=CH—, X may additionally be a hydrogen atom;

(c) 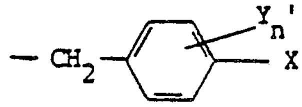

where X, n' and Y are as defined above;
(d) a group selected from

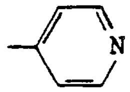 , 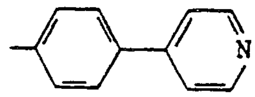

and 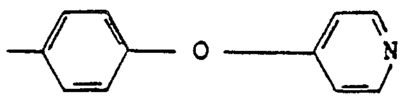 ; and

(e) a group selected from

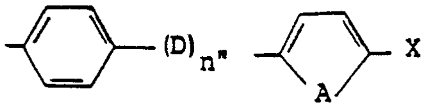 and 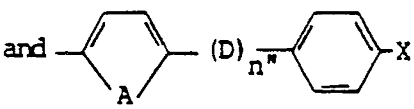

where X and n'' are as defined above, A ia an oxygen or sulphur atom and D' is an oxygen atom or a —$CH_2$— group.

The compounds of formula V above are prepared by processes directly analogous to those described for compounds of formula I above (e.g. in US Patent No. 3,957,824 and Shadbolt *et al* J. C. S. Perkin 1, (1976) No. 11, pages 1190 to 1195).

It has now surprisingly been found possible that compounds of the above formulae I and V may be prepared in enhanced yield and at temperatures below those heretofore employed.

According to the present invention there is provided a process for preparing a 4-hydroxy coumarin derivative of formula

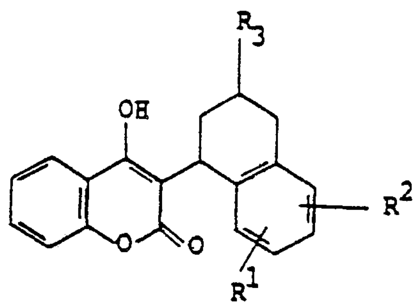

(I)

wherein $R^1$ and $R^2$ are independently selected from the group consisting of hydrogen, halogen, alkyl of up to 6 carbon atoms and alkoxy of up to 6 carbon atoms, and $R^3$ represents a group selected from

(a) 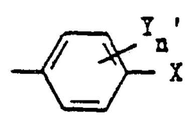

where X represents a halogen atom or a group $C_{1-12}$ alkyl, $C_{1-12}$alkoxy, $C_{3-8}$ cycloalkyl, CN, $NO_2$, $SO_2R^5$, $CF_3$, $OCF_3$, $COOR^5$ or $COR^5$, where $R^5$ is $C_{1-6}$ alkyl, n' is 0, 1 or 2 and Y is a fluorine or chlorine atom;

(b) 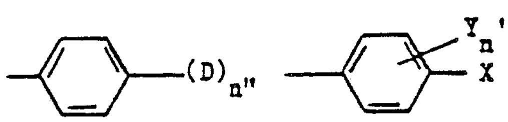

where n' and Y are as defined above, X is as defined above or is hydrogen, n'' is 0 or 1 and D is an oxygen atom or a group —O—$(CH_2)_m$—, —$(CH_2)_m$—O—, —O—$(CH_2)_m$—O—, —$(CH_2)_m$—O—$(CH_2)_p$—, —$(CH_2)$—$_m$, or —CH=CH—, or a sulphur analogue thereof, wherein m is in the range 1 to 6 and p is in the range 1 to 6;

(c) 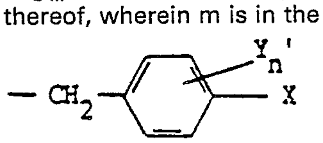

where X, n′ and Y are as defined in (a) above:

(d) a group selected from

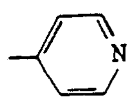 , 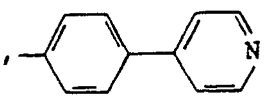

and 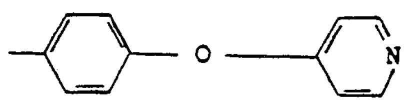 ; and

(e) a group selected from

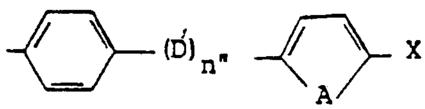 and 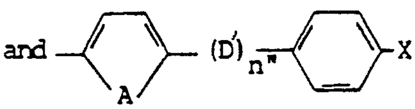

where X and n″ are as defined in (b) above, A is an oxygen or sulphur atom and D′ is an oxygen atom or a —$CH_2$— group, which process comprises condensing 4-hydroxycoumarin with a compound of formula

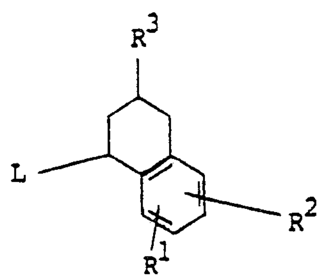

(VI)

or the corresponding 1,2-dihydronaphthalene, wherein $R^1$, $R^2$ and $R^3$ are as defined above, and L is a hydroxy group or a halogen atom, in the presence of an organic solvent and a catalyst characterised in that the solvent is selected from formic acid, a mixture of formic acid and at least one $C_{2-6}$ aliphatic carboxylic acid having a boiling temperature at atmospheric pressure in the range 60°C to 105°C and a liquid chlorinated alkane having a boiling temperature in the range 60°C to 125°C, and reaction is effected at a temperature in the range 60°C to the reflux temperature of the reaction mixture.

L in formula VI is preferably OH, Br or Cl.

Preferably in formula VI, $R^1$ and $R^2$ are both hydrogen.

When X is not hydrogen it preferably represents a fluorine, chlorine or bromine atom or a group CN, $NO_2$, $CF_3$ or $OCF_3$, more preferably a chlorine or bromine atom or a group CN or $CF_3$.

$R^3$ preferably has the formula

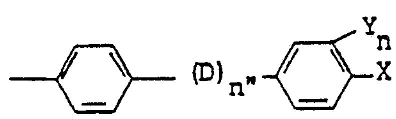

and it is preferred for D to be an oxygen atom, a group —O—$CH_2$—, —$CH_2$—$CH_2$—, —$CH_2$—$CH_2$—$CH_2$— or —CH=CH—. It is further preferred that n′ is 0, n″ is 0 or 1, D is an oxygen atom or a group —O—$CH_2$— and X is a group $CF_3$, H, CN or Br.

The process of the invention is of most particular interest when applied to the preparation of difenacoum, bromdifacoum or 3-[3-(4-(4-trifluoromethylbenzyloxy)phenyl)-1,2,3,4-tetrahydro-1-naphthyl]-4-hydroxycoumarin.

The organic solvent may be formic acid or a mixture of formic acid and at least one $C_{2-6}$ aliphatic carboxylic acid having a boiling temperature at atmospheric pressure in the range 60°C to 105°C. Mixtures of formic acid (boiling temperature 100.5°C) and glacial acetic acid (boiling temperature 118°C) having boiling temperatures in this range, particularly in volume proportions formic acid:acetic acid of about 3:1, are preferred.

With such solvents the catalyst may conveniently be a mineral acid, such as sulphuric or, preferably, orthophosphoric acid; an acid salt, such as potassium hydrogen sulphate; a Lewis acid such as ferric chloride, zinc chloride or potassium chloride; or silica gel.

Reaction temperatures in such solvent/catalyst systems are preferably in the range 70°C to reflux temperature, more preferably 80°C to reflux temperature.

In a preferred process of the invention, the solvent is a liquid chlorinated alkane, the catalyst is a sulphonic acid, and reaction is effected at a temperature in the range 60°C to the reflux temperature of the reaction mixture.

The chlorinated alkane is one which is liquid at ambient temperature, e.g. $C_{2-4}$ di- and trichloroalkanes.

Examples of such chlorinated alkanes include 1,2-dichloroethane (b.p. 83°C to 84°C), 1,1,2-trichloroethane (b.p. 113°C to 114°C), 1,1,1-trichloroethane (b.p. 74°C to 75°C), 1,1-dichloropropane (b.p. 87°C), 1,2-dichloropropane (b.p. 95°C to 96°C) and 1,3-dichloropropane (b.p. 125°C). 1,2-Dichloroethane, 1,1,2-trichloroethane and 1,3-dichloropropane have been found to be very suitable.

Examples of sulphonic acids include methanesulphonic acid, trifluoromethanesulphonic acid, and aryl sulphonic acids such as benzenesulphonic acid and *p*-toluenesulphonic acid.

In processes of the invention, the relative quantities of the reactants are not critical. However it is preferred for there to be 1 to 4 mols preferably 1 to 3 mols, of 4-hydroxycoumarin per mol of compound of formula VI, and, in the case of a sulphonic acid catalyst, for there to be 0.25 to 2 mols, preferably 0.5 to 1.5 mols, of sulphonic acid per mol of compound of formula VI.

The invention will be further understood from the following Examples.

## Example 1

3-(4-(4-Trifluoromethylbenzyloxy)phenyl)-1,2,3,4-tetrahydro-1-naphthol (8 g, 0.02 M), 4-hydroxycoumarin (6.5 g, 0.04 M) and *p*-toluenesulphonic acid (1.9 g, 0.01 M) were refluxed in 1,2-dichloroethane (80 ml) in a Dean-Stark apparatus under nitrogen, for 24 hours (boiling point of dichloroethane 83—84°C). The resulting solution was then cooled, washed with water and evaporated to yield a solid residue which was purified by chromatography on silica gel using dichloromethane as eluant to give 3-[3-(4-(4-trifluoromethylbenzyloxy)phenyl)-1,2,3,4-tetrahydro-1-naphthyl]-4-hydroxycoumarin (9.9 g, 90%) m.p. 85—86°C.

## Example 2

1-Chloro-3-(4-(4-trifluoromethylbenzyloxy)phenyl)-1,2,3,4-tetrahydronaphthalene (2.08 g, 0.005M) and 4-hydroxycoumarin (2.49 g, 0.015M) were added to a mixture of formic acid and glacial acetic acid (3:1 v/v; 8 ml) containing orthophosphoric acid (100 mg). The mixture was refluxed with stirring under nitrogen, for 3 hours. The resulting mixture was cooled and diluted with dichloromethane to give a solution which was washed with water. Evaporation of the dichloromethane solution gave a solid residue which was purified by chromatography on silica gel using dichloromethane as eluant to give 3-[3-(4-(4-trifluoromethylbenzyloxy)phenyl)-1,2,3,4-tetrahydro-1-naphthyl]-4-hydroxycoumarin (1.6 g 60%) m.p. 85—86°C.

## Examples 3 to 14

3-[3-(4-(4-Trifluoromethylbenzyloxy)phenyl)-1,2,3,4-tetrahydro-1-naphthyl]-4-hydroxycoumarin was prepared by process variants of Example 1, the volume of solvent in Examples 8 to 14 being higher (130 ml). The variants and the yields are given in Table I following, in which S.M. stands for starting material, "hydroxy" being the naphthol derivative used in Example 1 and "chloro" being the 1-chloronaphthalene derivative used in Example 2, pTSA is *p*-toluenesulphonic acid, BSA is benzenesulphonic acid, MSA is methanesulphonic acid, DCE is 1,2-dichloroethane (b.p. 83—84°C), TCE is 1,1,2-trichloroethane (b.p. 113—114°C) and DCP is 1,3-dichloropropane (b.p. 125°C).

Table I

| Example | S.M. | Reaction Temperature | Reaction Time (hours) | Mols 4-hydroxycoumarin per mol S.M. | Sulphonic acid (Mols acid per mol. S.M.) | Solvent | Yield |
|---|---|---|---|---|---|---|---|
| 3 | hydroxy | reflux | 22 | 3 | pTSA (1) | DCE | 87 |
| 4 | chloro | reflux | 24 | 3 | pTSA (1) | DCE | 74 |
| 5 | hydroxy | reflux | 24 | 3 | pTSA (0.5) | DCE | 82 |
| 6 | hydroxy | reflux | 24 | 3 | pTSA (1.5) | DCE | 82 |
| 7 | hydroxy | reflux | 24 | 3 | pTSA (0.5) | DCE | 87 |
| 8 | hydroxy | reflux | 26 | 1.05 | pTSA (0.5) | DCE | 88 |
| 9 | hydroxy | reflux | 25 | 2 | pTSA (0.5) | DCE | 92.25 |
| 10 | hydroxy | reflux | 25 | 1 | pTSA (0.5) | DCE | 90 |
| 11 | hydroxy | reflux | 26 | 2 | pTSA (0.5) | TCE | 90 |
| 12 | hydroxy | reflux | 26 | 2 | pTSA (0.5) | DCP | 90 |
| 13 | hydroxy | reflux | 24 | 2 | MSA (0.5) | DCE | 92 |
| 14 | hydroxy | reflux | 24 | 2 | BSA (0.5) | DCE | 92.5 |

Example 15

3-(4'-Bromobiphenyl-4-yl)-1,2,3,4-tetrahydro-1-naphthol (7.56 g, 0.02M), 4-hydroxycoumarin (6.5 g, 0.04M) and *p*-toluenesulphonic acid (1.9 g 0.01M) were refluxed in 1,2-dichloroethane (130 ml) in a Dean-Stark apparatus for 24 hours. The resulting solution was cooled, dichloromethane (150 ml) was added, the solution was washed with 5% w/v aqueous sodium carbonate (2 × 100 ml) and with water (150 ml), dried ($MgSO_4$) and evaporated to yield a solid residue which was purified by chromatography on silica gel using dichloromethane as eluent to give brodifacoum, 3-[3-(4'-bromobiphenyl-4-yl)-1,2,3,4-tetrahydro-1-naphthyl]-4-hydroxycoumarin, (9.7 g, 93%) mp 228—235°C.

Example 16

The process of Example 15 was followed except that 1,1,2-trichloroethane (20 ml) was used in place of the 1,2-dichloroethane, and the reaction mixture was refluxed for 6 hours (not 24 hours). Brodifacoum (8.8g, 84%) mp 228—235°C was obtained.

Example 17

The process of Example 15 was followed except that 3-biphenyl-4-yl-1,2,3,4-tetrahydro-1-naphthol was used in place of the bromo compound, the resulting product being difenacoum, 3-(3-biphenyl-4-yl-1,2,3,4-tetrahydro-1-naphthyl)-4-hydroxycoumarin, mp 215—219°C, in 95% yield.

Example 18

The process modifications of Example 16 were applied to the process of Example 17. Difenacoum, mp 215—219°C, was obtained in 83% yield.

Example 19

The process of Example 17 was followed except that in place of the naphthol was employed 2-biphenyl-4-yl-1,2-dihydronaphthalene. Difenacoum, mp 215—219°C, was obtained in 76% yield.

Comparative Example A

(in accordance with GB—A—2 126 578, page 10 lines 44 to page 11 line 2 and page 7 lines 55 to 59).

A mixture of 3(4-(4-trifluoromethylbenzyloxy)phenyl)-1,2,3,4-tetrahydro-1-naphthol (3.9 g, 9.8 mmol) and 4-hydroxycoumarin (1.8 g, 11.1 mmol) was heated at 160—170°C for one hour. After cooling to ambient temperature, the resulting mixture was purified by chromatography on silica gel using dichloromethane as eluant, to give 3-[3-(4-(4-trifluormethylbenzyloxy)-phenyl)-1,2,3,4-tetrahydro-1-naphthyl]-4-hydroxycoumarin (2,35 g, 44%) m.p. 85—86°C.

## Claims

1. A process for preparing a 4-hydroxycoumarin derivative of formula

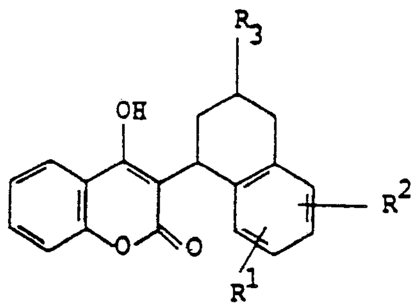

(I)

wherein $R^1$ and $R^2$ are independently selected from the group consisting of hydrogen, halogen, alkyl of up to 6 carbon atoms and alkoxy of up to 6 carbon atoms, and $R^3$ represents a group selected from

(a) 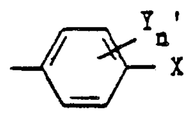

where X represents a halogen atom or a group $C_{1-12}$ alkyl, $C_{1-12}$ alkoxy, $C_{3-8}$ cycloalkyl, CN, $NO_2$, $SO_2R^5$, $CF_3$, $OCF_3$, $COOR^5$ or $COR^5$, where $R^5$ is a $C_{1-6}$ alkyl, n′ is 0, 1 or 2 and Y is a fluorine or chlorine atom;

(b) 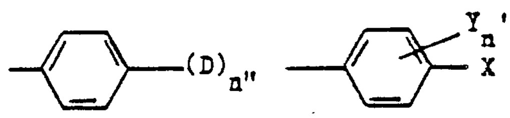

where n′ and Y are as defined above, X is as defined above or is hydrogen, n″ is 0 or 1 and D is an oxygen atom or a group $-O-(CH_2)_m-$, $-O-(CH_2)_m-O-$, $-O-(CH_2)_m-O-$, $-O-(CH_2)_m-O-(CH_2)_p$, $(CH_2)-_m$, or $-CH=CH-$, or a sulphur analogue thereof, wherein m is in the range 1 to 6 and p is in the range 1 to 6;

(c) 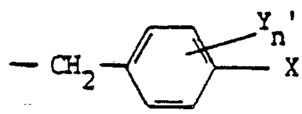

where X, n′ and Y are as defined in (a) above:
(d) a group selected from

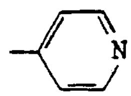 , 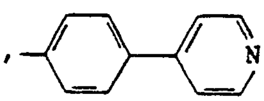

and 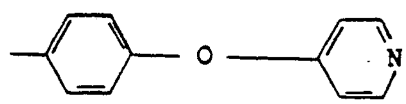 ; and

(e) a group selected from

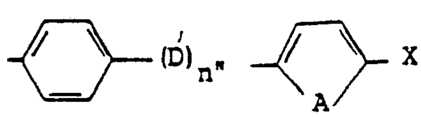 and 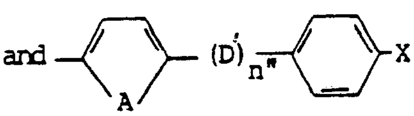

where X and n″ are as defined in (b) above, A is an oxygen or sulphur atom and D′ is an oxygen atom or a $-CH_2-$ group, which process comprises condensing 4-hydroxycoumarin with a compound of formula

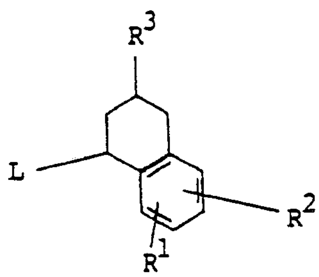

(VI)

or the corresponding 1,2-dihydronaphthalene, wherein $R^1$, $R^2$ and $R^3$ are as defined above, and L is a hydroxy group or a halogen atom, in the presence of an organic solvent and a catalyst characterised in that the solvent is selected from formic acid, a mixture of formic acid and at least one $C_{2-6}$ aliphatic carboxylic acid having a boiling temperature at atmospheric pressure in the range 60°C to 105°C and a liquid chlorinated alkane having a boiling temperature in the range 60°C to 125°C, and reaction is effected at a temperature in the range 60°C to the reflux temperature of the reaction mixture.

2. A process according to claim 1, wherein $R^1$ and $R^2$ are both hydrogen.

3. A process according to claim 1 or 2 wherein X is a fluorine, chlorine or bromine atom or a group CN, $NO_2$, $CF_3$ or $OCF_3$.

4. A process according to claim 3 wherein X is a chlorine or bromine atom or a group CN or $CF_3$.

5. A process according to claim 1 or 2 wherein $R^3$ represents a group

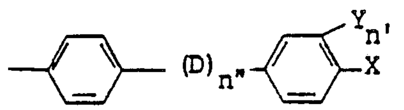

wherein n', Y, n'' and D are as defined in claim 1 and X is hydrogen or is as defined in claim 3 or 4.

6. A process according to claim 5 wherein $R^3$ represents a group

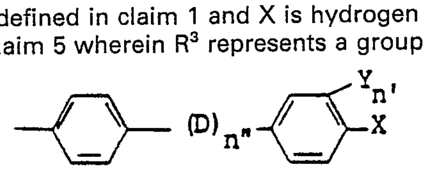

wherein X, n' Y and n'' are as defined in claim 5 and D is an oxygen atom, a group $—O—CH_2—$, $—CH_2—CH_2—$, $—CH_2—CH_2—CH_2—$ or $—CH{=}CH—$.

7. A process according to claim 6 wherein n' is 0, n'' is 0 or 1, D is an oxygen atom or a group $—O—CH_2—$ and X is a group $CF_3$, H, CN, or Br.

8. A process according to any of claims 1 to 7, wherein the solvent is a liquid chlorinated alkane the catalyst is a sulphonic acid, and reaction is effected at a temperature in the range 60°C to the reflux temperature of the reaction mixture.

9. A process according to claim 8 wherein the liquid chlorinated alkane is selected from $C_{2-4}$ di- and trichloroalkanes.

10. A process according to claim 9 wherein the liquid chlorinated alkane is selected from 1,2-dichloroethane, 1,1,2-trichloroethane, and 1,3-dichloropropane.

11. A process according to any of claims 1 to 10 wherein the catalyst is selected from methanesulphonic acid, benzenesulphonic acid and p-toluenesulfonic acid.

**Patentansprüche**

1. Verfahren zur Herstellung eines 4-Hydroxycumarinderivats der Formel

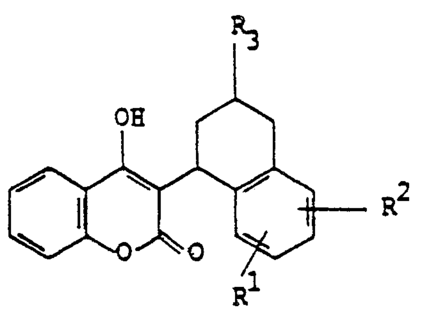

(I)

worin $R^1$ und $R^2$ unabhängig voneinander aus der aus Wasserstoff, Halogen, Alkyl mit bis zu 6 Kohlenstoffatomen und Alkoxy mit bis zu 6 Kohlenstoffatomen bestehenden Gruppe ausgewählt sind und $R^3$ eine Gruppe bedeutet, ausgewählt unter

(a)

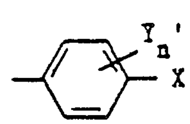

worin X ein Halogenatom oder eine $C_{1-12}$Alkylgruppe, $C_{1-12}$Alkoxygruppe, $C_{3-8}$Cycloalkylgruppe, CN, $NO_2$, $SO_2R^5$, $CF_3$, $COOR^5$ oder $COR^5$ darstellt, worin $R^5$ für $C_{1-6}$Alkyl steht, n' den Wert 0, 1 oder 2 hat und Y ein Fluor- oder Chlolatom ist;

(b) 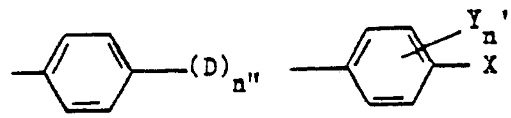

worin n′ und Y wie vorstehend definiert sind, X wie oben definiert ist oder Wasserstoff bedeutet, n″ den Wert 0 oder 1 hat und D ein Sauerstoffatom oder eine Gruppe $—O—(CH_2)_m—$, $(CH_2)_m—O—$, $—O—(CH_2)_m—O—$, $—(CH_2)_m—O—(CH_2)_p$, $—(CH_2)—_m$, oder $—CH{=}CH—$, oder ein Schwefelanalogon hievon, bedeutet, worin m im Bereich 1 bis 6 liegt und p im Bereich 1 bis 6 liegt;

(c) 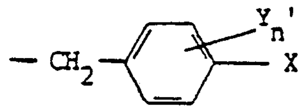

worin X, n′ und Y wie oben unter (a) definiert sind;

(d) einer Gruppe, ausgewählt unter

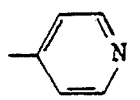 , 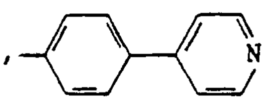

und 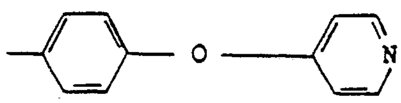 ; und

(e) einer Gruppe, ausgewählt unter

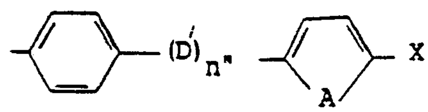 und 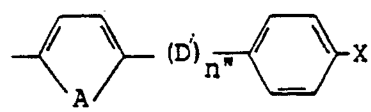

worin X und n″ sie oben unter (b) definiert sind, A ein Sauerstoff- oder Schwefelatom ist und D′ ein Sauerstoffatom oder eine $—CH_2$-Gruppe darstellt, welches Verfahren ein Kondensieren von 4-Hydroxycumarin mit einer Verbindung der Formel

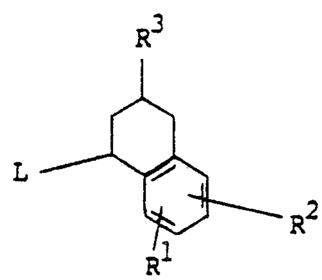

(VI)

oder dem entsprechenden 1,2-Dihydronaphthalin umfaßt, worin $R^1$, $R^2$ und $R^3$ wie oben definiert sind un L ein Hydroxygruppe oder ein Halogenatom darstellt, in Anwesenheit eines organischen Lösungsmittels und eines Katalysators, dadurch gekennzeichnet, daß das Lösungsmittel unter Ameisensäure, einem Gemisch aus Ameisensäure und wenigstens einer $C_{2-6}$ aliphatischen Carbonsäure mit einer Siedetemperatur bei Atmosphärendruck im bereich 60°C bis 105°C und einem flüssigen chlorierten Alkan mit einer Siedetemperatur im Bereich 60°C bis 125°C ausgewählt wird und daß die Umsetzung bei einer Temperatur im Bereich 60°C bis zur Rückflußtemperatur des Reaktionsgemisches vorgenommen wird.

2. Verfahren nach Anspruch 1, worin $R^1$ und $R^2$ jeweils Wasserstoff bedeuten.

3. Verfahren nach Anspruch 1 oder 2, worin X ein Fluor-, Chlor- oder Bromatom oder eine Gruppe CN, $NO_2$, $CF_3$ oder $OCF_3$ bedeutet.

4. Verfahren nach Anspruch 3, worin X ein Chlor- oder Bromatom oder eine Gruppe CN oder $CF_3$ bedeutet.

5. Verfahren nach Anspruch 1 oder 2, worin $R^3$ eine Gruppe

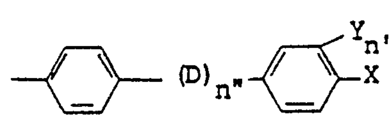

darstellt, worin n', Y, n'' und D wie in Anspruch 1 definiet sind und X Wasserstoff bedeutet oder wie in Anspruch 3 oder 4 definiert ist.

6. Verfahren nach Anspruch 5, worin $R^3$ eine Gruppe

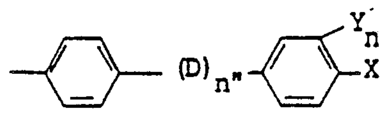

darstellt, worin X, n', Y und n'' wie in Anspruch 5 definiert sind und D ein Sauerstoffatom oder eine Gruppe $—O—CH_2—$, $—CH_2—CH_2—$, $—CH_2—CH_2—CH_2—$ oder $—CH=CH—$ bedeutet.

7. Verfahren nach Anspruch 6, worin n den Wert 0 hat, n'' den Wert 0 oder 1 hat, D ein Sauerstoffatom oder ein Gruppe $—O—CH_2—$ darstellt und X für eine Gruppe $CF_3$, H, CN oder Br steht.

8. Verfahren nach einem der Ansprüche 1 bis 7, worin das Lösungsmittel ein flüssiges chloriertes Alkan ist, der Katalysator ein Sulfonsäure ist und die Umsetzung bei einer Temperatur im Bereich von 60°C bis zur Rückflußtemperatur des Reaktionsgemisches ausgeführt wird.

9. Verfahren nach Anspruch 8, worin das flüssige chlorierte Alkan unter $C_{2-4}$Di- und Trichloralkanen ausgewählt wird.

10. Verfahren nach Anspruch 9, worin das flüssige chlorierte Alkan unter 1,2-Dichlorethan, 1,1,2-Trichlorethan und 1,3-Dichlorpropan ausgewählt wird.

11. Verfahren nach einem der Ansprüche 1 bis 10, worin der Katalysator unter Methansulfonsäure, Benzosulfonsäure und p-Toluolsulfonsäure ausgewählt wird.

**Revendications**

1. Un procédé pour la préparation d'un dérivé de 4-hydroxycoumarine de formule

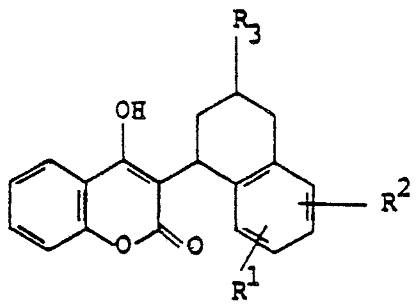

(I)

où $R^1$ et $R^2$ sont choisis indépendamment dans le groupe constitué par l'hydrogène, les halogènes et les radicaux alcoyle ayant jusqu'à 6 atomes de carbone et alcoxy ayant jusqu'à 6 atomes de carbone, et $R^3$ représente un groupe choisi parmi

(a)

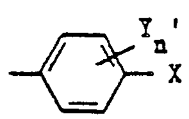

où X représente un atome d'halogène ou un groupe alcoyle en $C_{1-12}$, alcoxy en $C_{1-12}$, cycloalkoyle en $C_{3-8}$, CN, $NO_2$, $SO_2R^5$, $CF_3$, $CCF_3$, $COOR^5$ ou $COR^5$, où $R^5$ est un groupe alcoyle en $C_{1-6}$, n' est 0, 1 ou 2 et Y est un atome de fluor ou de chlore;

(b)

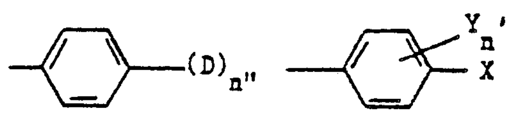

où n' et Y sont tels que définis ci-dessus, X est tel que défini ci-dessus ou est de l'hydrogène, n'' est 0 ou 1 et D est un atome d'oxygène ou un groupe $—O—(CH_2)_m—$, $—(CH_2)_m—O—$, $—(CH_2)_m—O—$, $—(CH_2)_m—O—(CH_2)_p$, $—(CH_2)—_m$, ou $—CH=CH—$, ou un analogue sulfuré correspondant, où m va de 1 à 6 et p va de 1 6;

(c)

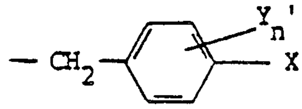

où X, n' et Y sont tels que définis en (a) ci-dessus;

(d) un groupe choisi parmi

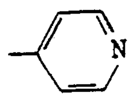 , 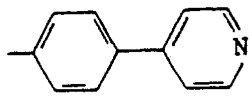

et 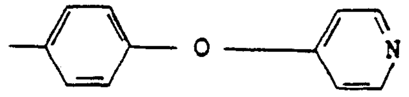 ; et

(e) un groupe choisi parmi

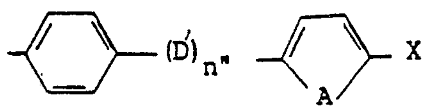 et 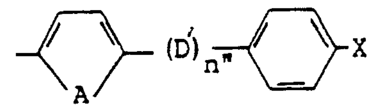

où X et n'' sont tels que définis en (b) ci-dessus, A est un atome d'oxygène ou de soufre et D' est un atome d'oxygène ou un groupe —$CH_2$—, lequel procédé comprend la condensation de 4-hydroxycoumarine avec un composé de formule

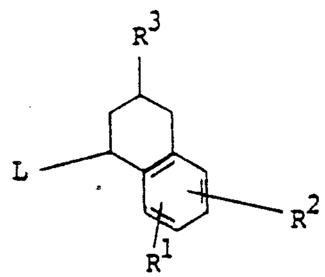

(VI)

ou le 1,2-dihydronaphtalène correspondant, où $R^1$, $R^2$ et $R^3$ sont dtels que définis ci-dessus et L est un groupe hydroxy ou un atome d'halogène, en présence d'un solvant organique et d'un catalyseur, caractérisé en ce que le solvant est choisi parmi l'acide formique, un mélange d'acide formique et d'au moins un acide carboxylique aliphatique en $C_{2-6}$ ayant une température d'ébullition à la pression atmosphérique comprise dans l'intervalle de 60°C à 105°C et un alcane chloré liquide ayant une température d'ébullition comprise dans l'intervalle de 60°C à 125°C et que la réaction est conduite à une température comprise dans l'intervalle de 60°C à la température de reflux du mélange réactionnel.

2. Un procédé selon la revendication 1, dans lequel $R^1$ et $R^2$ sont tous deux de l'hydrogène.

3. Un procédé selon la revendication 1 ou 2, dans lequel X est un atome de fluor, de chlore ou de brome ou un groupe CN, $NO_2$, $CF_3$ ou $CCF_3$.

4. Un procédé selon la revendication 3, dans lequel X est un atome de chlore ou de brome ou un groupe CN ou $CF_3$.

5. Un procédé selon la revendication 1 ou 2, dans lequel $R^3$ représente un groupe

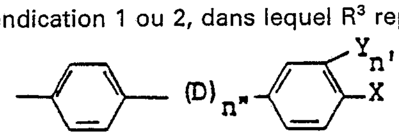

où n', Y, n'' et D sont tels que définis dans la revendication 1 et X est de l'hydrogène ou est tel que défini dans la revendication 3 ou 4.

6. Un procédé selon la revendication 5, dans lequel $R^3$ représente un groupe

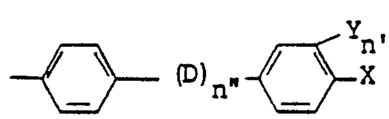

où X, n', Y et n'' sont tels que définis dans la revendication 5 et D est un atome d'oxygène, un groupe —O—$CH_2$—, —$CH_2$—$CH_2$—, —$CH_2$—$CH_2$—$CH_2$— ou —CH=CH—.

7. Un procédé selon la revendication 6, dans lequel n' est 0, n'' eswt 0 ou 1, D est un atome d'oxygène ou un groupe —O—$CH_2$— et X est un groupe $CF_3$, H, CN ou Br.

8. Un procédé selon l'une quelconque des revendications 1 à 7, dans lequel le solvant est un alcane chloré liquide, le catalyseur est un acide sulfonique et la réaction est conduite à une température comprise dans l'intervalle de 60°C à la température de reflux du mélange réactionnel.

9. Un procédé selon la revendication 8, dans lequel l'alcane chloré liquide est choisi parmi les diet trichloroalcanes en $C_{2-4}$.

10. Un procédé selon la revendication 9, caractérisé en ce que l'alcane chloré liquide est choisi parmi le 1,2-dichloroéthane, le 1,1,2-trichloroéthane et le 1,3-dichloropropane.

11. Un procédé selon l'une quelconque des revendications 1 à 10, dans lequel le catalyseur est choisi parmi l'acide méthanesulfonique, l'acide benzènesulfonique et l'acide *p*-toluènesulfonique.